(19) Europäisches Patentamt
European Patent Office
Office européen des brevets

(11) **EP 4 647 404 A1**

(12) **EUROPÄISCHE PATENTANMELDUNG**

(43) Veröffentlichungstag:
**12.11.2025 Patentblatt 2025/46**

(21) Anmeldenummer: **25174539.4**

(22) Anmeldetag: **06.05.2025**

(51) Internationale Patentklassifikation (IPC):
**C02F 1/00** *(2023.01)* **C02F 1/44** *(2023.01)*
**C02F 1/68** *(2023.01)* **B01D 61/02** *(2006.01)*
**B01D 61/12** *(2006.01)* **B01D 65/02** *(2006.01)*
**C02F 1/72** *(2023.01)*

(52) Gemeinsame Patentklassifikation (CPC):
**C02F 1/441; B01D 61/12; B01D 65/06; C02F 1/008;**
**C02F 1/686;** B01D 61/025; B01D 2321/02;
B01D 2321/16; B01D 2321/40; C02F 1/72;
C02F 1/722; C02F 2209/005; C02F 2209/02;
C02F 2209/03; C02F 2209/06; (Forts.)

(84) Benannte Vertragsstaaten:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Benannte Erstreckungsstaaten:
**BA**
Benannte Validierungsstaaten:
**GE KH LA MA MD TN**

(30) Priorität: **07.05.2024 DE 102024112730**

(71) Anmelder: **B. Braun Avitum AG**
**34212 Melsungen (DE)**

(72) Erfinder:
• **LIEB, Nino**
**34117 Kassel (DE)**
• **ODERNHEIMER, Philipp**
**34212 Melsungen (DE)**

(74) Vertreter: **Tergau & Walkenhorst**
**Intellectual Property GmbH**
**Lurgiallee 12**
**60439 Frankfurt am Main (DE)**

(54) **VERFAHREN ZUR CHEMISCHEN DESINFEKTION EINER UMKEHROSMOSEANLAGE UND UMKEHROSMOSEANLAGE**

(57) Verfahren zur chemischen Desinfektion einer Umkehrosmoseanlage (109), welche einen Vorlagetank (102), eine Ringleitung (110) zum Anschluss von Verbrauchern (111), wenigstens ein Membranmodul (105), eine Zulaufleitung (124) zum Membranmodul (105), wenigstens eine Druckpumpe (104) zum Pumpen von Flüssigkeit in das Membranmodul (105) sowie einen Desinfektionsmittelbehälter (123) umfasst, wobei Desinfektionsmittel aus dem Desinfektionsmittelbehälter (123) in den Vorlagetank (102) gefördert wird. Das Verfahren umfasst die Schritte

- Festsetzen einer Zielkonzentration des Desinfektionsmittels;

- Bestimmen der aufgrund der Zielkonzentration des Desinfektionsmittels benötigten Menge des Desinfektionsmittels;

- Ausleiten der für die Zielkonzentration benötigten Menge des Desinfektionsmittels aus dem Desinfektionsmittelbehälter (123) in den Vorlagetank (102) durch eine Ausleitungsvorrichtung (112, 122).

FIG. 1

(52) Gemeinsame Patentklassifikation (CPC): (Forts.)
C02F 2209/40; C02F 2303/04; C02F 2303/16;
C02F 2303/20

**Beschreibung**

Bei der chemischen Desinfektion von Umkehrosmoseanlagen zur Produktion von Permeat für die Dialysetherapie müssen gewöhnlich die Desinfektionsmittelmengen vom Nutzer manuell berechnet und anschließend in das System gefüllt werden. Außerdem sind die Systeme nicht in der Lage die Desinfektionsmittelmengen während des laufenden Betriebs zu regeln.

Aktuell werden bei einer chemischen Desinfektion alle Dialysegeräte von der Ringleitung getrennt und optional die Frischwasserversorgung ab einem bestimmten Prozessschritt abgeschaltet. Dadurch können nur die Umkehrosmoseanlage und die Ringleitung chemisch desinfiziert werden. Das Desinfektionsmittel wird dabei entweder von einer Person in den Tank der Umkehrosmoseanlage geschüttet oder automatisch vom Gerät angesaugt.

Aus der DE 195 38 818 A1 ist eine Anlage zur Versorgung einer Dialysestation mit Dialysewasser bekannt, mit einer Ringleitung, an welche die Dialysestation angeschlossen ist, und mit einem Umkehrosmosemodul zur Herstellung des Dialysewassers, wobei das vom Umkehrosmosemodul aus einem Rohwasseranschluss hergestellte Permeat als Dialysewasser über ein Vorlaufventil in die Ringleitung einleitbar ist. Dabei ist eine Impfmittelleitung zum Zuführen von chemischen Reinigungsmitteln an die Ringleitung angeschlossen, wobei in der Impfmittelleitung eine Dosierpumpe angeordnet ist.

Aus der DE 10 2006 026 107 B3 ist eine Vorrichtung zur Desinfektion einer Umkehrosmoseanlage mit einer Pumpe bekannt, die das über eine Saugleitung aus einem atmosphärisch belüfteten Vorratsbehälter entnommene chemische Desinfektionsmittel in das Leitungssystem der Umkehrosmoseanlage fördert. Die Umkehrosmoseanlage weist eine in die Saugleitung eingefügte Ansaugkammer mit einem oberen Anschluss zu der Pumpe und einem unteren Anschluss zu dem Vorratsbehälter sowie mit einer mit dem oberen Teil verbundenen Belüftungsleitung auf, die mit einem Absperrorgan versehen ist, das im Betriebszustand der Desinfektionsmittelzufuhr geschlossen und während der übrigen Betriebszustände der Umkehrosmoseanlage geöffnet ist.

Nachteilig bei bekannten Verfahren ist, dass die im Zentrum befindlichen Dialysegeräte ebenfalls zentral mit Desinfektionsmittel versorgt werden oder dass dezentrale Kanister mit Desinfektionsmitteln von Nutzern eingesetzt werden müssen. Ebenfalls muss der Nutzer das zu verwendende Desinfektionsmittel manuell einfüllen oder ein zu füllendes Volumen bestimmen.

Daneben zeigt die Praxis, dass die ausführenden Personen aufgrund von Unsicherheit über die einzufüllende Menge an Desinfektionsmittel eine Überkonzentration herbeiführen. Dies verschwendet zum einen Chemikalien und kann unter Umständen zu einem unerlaubten pH-Wert innerhalb der Anlage führen. Alternativ kann durch fehlerhafte Bestimmung/Berechnung der Desinfektionsmittelmenge eine Unterkonzentration erfolgen, welche unter Umständen zu einer unvollständigen Desinfektion führt. Zwar kann eine Überkonzentration zu Schäden für die Anlage führen, allerdings führt die Gefahr einer potenziellen Unterkonzentration zu dem Erzeugen einer Überkonzentration durch den Nutzer.

Der Erfindung liegt daher die Aufgabe zugrunde, ein verbessertes Verfahren zur chemischen Desinfektion einer Umkehrosmoseanlage bereitzustellen, welches die Nachteile des Standes der Technik überwindet. Insbesondere soll dadurch die Genauigkeit einer gewünschten initialen Zielkonzentration des Desinfektionsmittels erhöht werden.

In Bezug auf das Verfahren wird diese Aufgabe erfindungsgemäß gelöst durch ein Verfahren mit den Merkmalen von Anspruch 1. Das Verfahren zur chemischen Desinfektion einer Umkehrosmoseanlage, welche einen Vorlagetank, eine Ringleitung zum Anschluss von Verbrauchern, wenigstens ein Membranmodul, eine Zulaufleitung zum Membranmodul, wenigstens eine Druckpumpe zum Pumpen von Flüssigkeit in das Membranmodul sowie einen Desinfektionsmittelbehälter umfasst, wobei das Desinfektionsmittel aus dem Desinfektionsmittelbehälter in den Vorlagetank gefördert wird, umfasst die Schritte des Festsetzen einer Zielkonzentration des Desinfektionsmittels, des Bestimmens der aufgrund einer Zielkonzentration des Desinfektionsmittels benötigten Menge des Desinfektionsmittels und des Ausleitens der für die Zielkonzentration benötigten Menge des Desinfektionsmittels aus dem Desinfektionsmittelbehälter in den Vorlagetank durch eine Ausleitungsvorrichtung.

Vorteilhafte Ausgestaltungen der Erfindung sind Gegenstand der Unteransprüche.

Die Erfindung geht von der Überlegung aus, dass für eine zuverlässige chemische Desinfektion einer Umkehrosmoseanlage die vorgegebene Zielkonzentration des Desinfektionsmittels möglichst genau realisiert und eingehalten werden sollte. Während eine zu geringe Konzentration an Desinfektionsmittel den Erfolg der Desinfizierung gefährdet, kann eine zu hohe Menge aufgrund des zu hohen pH-Wertes zu Bauteilschädigungen in der Umkehrosmoseanlage bzw. den angeschlossenen Verbrauchern führen.

Die genaue Realisierung der Zielkonzentration ist nicht immer gewährleistet, wenn das Desinfektionsmittel von einem Nutzer von Hand abgefüllt wird. Auch kann in dem chemischen Verfahren nicht auf eine Änderung des Desinfektionsgemisches, d.h. dem Gemisch aus Prozesswasser und Desinfektionsmittel, reagiert werden.

Wie nunmehr erkannt wurde, lässt sich eine genaue Realisierung der Zielkonzentration erreichen, indem die zur Realisierung der Zielkonzentration benötigte Menge an Desinfektionsmittel aus einem dafür vorgesehenen Desinfektionsmittelbehälter ausgeleitet wird.

Im Unterschied zu aus dem Stand der Technik bekannten Verfahren wird erfindungsgemäß die tatsächlich benötigte Menge an Desinfektionsmittel berechnet. Das gilt sowohl für das initiale Befüllen als auch für die Nachregelung. Es wird eine kontrollierte Desinfektion mit kontrollierten pH-Wert bzw. Konzentrationsverhältnissen erzielt.

Bei Verfahren nach dem Stand der Technik, bei denen so lange Desinfektionsmittel in den Kreislauf gepumpt, bis eine Leitfähigkeitsgrenze überschritten wird, liegt der Nachteil vor, dass die Konzentration in der Anlage großenteils unklar/ unbestimmt ist, da die notwendigen Parameter (Gesamtvolumen an Flüssigkeit in der Anlage) unbekannt sind.

Der Begriff "Desinfektionsmittel" bezeichnet das Mittel bzw. Konzentrat, welches im Desinfektionsmittelbehälter bereitgestellt wird, während der Begriff "Desinfektionsmittelgemisch" das Gemisch aus Desinfektionsmittel und Wasser bezeichnet.

Die Förderung von Desinfektionsmittel aus dem Desinfektionsmittelbehälter in den Vorlagetank erfolgt bevorzugt automatisch, kann aber auch manuell, d.h. durch einen Benutzer, erfolgen.

Der Desinfektionsmittelbehälter ist vorteilhafterweise ein Vorratstank, welcher mehr Desinfektionsmittel umfassen kann, als für eine geplante Desinfektion der Umkehrosmoseanlage mit einer vorgegebenen Zielkonzentration des Desinfektionsmittels benötigt wird. Dadurch wird sichergestellt, dass genügend Desinfektionsmittel zur Verfügung steht, auch wenn Verbraucher zeitgleich desinfiziert werden und sich die benötigte Menge an Desinfektionsgemisch im Laufe des Verfahrens vergrößert. Der Desinfektionsmittelbehälter kann auch als ein Kanister ausgebildet sein. Der Desinfektionsmittelbehälter bzw. Vorratstank kann ein Teil der Umkehrosmoseanlage sein oder er kann ein externer Kanister sein, welcher zur Umkehrosmoseanlage gestellt und mit ihr verbunden werden kann. In bevorzugter Weise wird das Desinfektionsmittel aus einem mitgelieferten Kanister abgepumpt.

Erfindungsgemäß weist das Verfahren die Schritte des Überwachens und Bestimmens der aktuellen Konzentration des Desinfektionsmittels und, wenn die aktuelle Konzentration sich von der Zielkonzentration im Rahmen eines Schwellenbereiches unterscheidet, des Nachförderns von Desinfektionsmittel und Prozesseingangswasser in den Vorlagetank entsprechend der Zielkonzentration auf. Auf diese Weise wird auf eine sich im Laufe der Zeit ändernde Gesamtmenge des Desinfektionsgemisches reagiert und die Menge des Desinfektionsgemisches kann im Laufe des Verfahrens angepasst werden. Dadurch wird ermöglicht, dass während der chemischen Desinfektion der Umkehrosmoseanlage auch angeschlossene Verbraucher, insbesondere Dialysegeräte, chemisch desinfiziert werden können.

Das Überwachen und Bestimmen der aktuellen Konzentration des Desinfektionsmittels wird erfindungsgemäß durch Bestimmen des Flüssigkeitsvolumens der Flüssigkeit im Vorlagetanks durchgeführt.

Das Bestimmen des Flüssigkeitsvolumens erfolgt bevorzugt durch eine Druckmessung mit einem Drucksensor. Hierbei wird eine Flüssigkeitsmenge einer entsprechenden Druckänderung zugeordnet, sodass bei der Messung einer Druckveränderung auf die Änderung der Flüssigkeitsmenge geschlossen werden kann. Das Bestimmen des Flüssigkeitsvolumens kann alternativ oder in Kombination auch mechanisch, (z. B. mittels eines Schwimmers), optisch (z. B. mittels einer Lichtschranke) und/oder elektrisch (z. B. mittels einer Leitfähigkeitsmessung) erfolgen.

Das Bestimmen des Flüssigkeitsvolumens erfolgt bevorzugt durch eine Volumenstrommessung wenigstens eines Volumenstromsensors. Der Volumenstromsensor misst dabei bevorzugt die Menge an Flüssigkeit, die aus der Umkehrosmoseanlage in die Ringleitung strömt. Auf diese Weise kann Flüssigkeit, welche von den Verbrauchern entnommen wird, detektiert werden. Es kann darüber hinaus mit einem Volumenstromsensor die Menge an Desinfektionsmittel, die den Desinfektionsmittelbehälter verlässt, bestimmt werden. Auf diese Weise ist eine Bestimmung der abgeflossenen Flüssigkeitsmengen aus Desinfektionsmittelbehälter und Vorlagetank durch Integration der Volumenströme unmittelbar möglich. Sofern nur ein Volumenstromsensor am Ringleitungsvorlauf vorgesehen ist (wie hier beschrieben), muss der Durchfluss, welcher in den Tank zurückströmt, bekannt und im Wesentlichen kontinuierlich sein, da ansonsten nicht bestimmt werden kann. wieviel Flüssigkeit den Kreislauf verlässt. In einer alternativen Ausführung kann ein zweiter Volumenstromsensor am Ringleitungsrücklauf angebracht werden, um die beiden Messwerte gegeneinander zu bilanzieren. Die Differenz der Sensorwerte ergibt dabei das verbrauchte Volumen.

Das Bestimmen der aktuellen Konzentration des Desinfektionsmittels kann bevorzugt mit wenigstens einem stoffselektiven Sensor durchgeführt werden. Die Stoffkonzentration kann auf diese Weise direkt gemessen werden und muss nicht errechnet werden. Dadurch wird die Genauigkeit der Konzentrationsbestimmung verbessert, da Systemeigenschaften, welche das Ergebnis verfälschen können, vernachlässigt werden können.

Der stoffselektive Sensor ist bevorzugt als amperometrischer Sensor ausgebildet. Der amperometrische Sensor reagiert dabei bevorzugt sensitiv auf Wasserstoffperoxid und/oder Peressigsäure und/oder Zitronensäure, welche gewöhnlich die Hauptbestandteile von modernen Desinfektionsmitteln sind.

Das Bestimmen der aktuellen Konzentration des Desinfektionsmittels kann bevorzugt durch eine pH-Wert-Messung durchgeführt werden. Dazu wird aus dem gemessenen pH-Wert die Molalität der Wasserstoffionen ermittelt. Mit der Molalität und der bekannten Zusammensetzung des Desinfektionsmittels kann dann eine Konzentration errechnet werden.

Das Nachfördern von Desinfektionsmittel und Prozesseingangswasser in den Vorlagetank erfolgt in einer ersten bevorzugten Ausführungsform kontinuierlich. Auf diese Weise wird ermöglicht, dass zu jedem Zeitpunkt möglichst genau die gewünschte Zielkonzentration des Desinfektionsmittels im Anlagenverbund, d.h. in der Umkehrosmoseanlage und der Ringleitung sowie ggf. den Verbrauchern, vorliegt.

In einer alternativen bevorzugten Ausführung erfolgt das Nachfördern, wenn die dem Vorlagetank entnommene Flüssigkeitsmenge einen Volumenschwellenwert überschreitet. Dies hat den Vorteil, dass die Messunsicherheiten sowie Trägheit von Ventilen und Pumpen besser beherrschbar sind.

In einer bevorzugten Ausführung des Verfahrens wird das Fördern von Desinfektionsmittel aus dem Desinfektionsmittelbehälter in den Vorlagetank mit einer Dosierpumpe durchgeführt, welche die Ausleitungsvorrichtung ist.

In einer alternativen bevorzugten Ausführung des Verfahrens erfolgt das Fördern von Desinfektionsmittel aus dem Desinfektionsmittelbehälter in den Vorlagetank durch Nutzung der Schwerkraft. Dazu ist im montierten Zustand bzw. Betriebszustand der Umkehrosmoseanlage für die chemische Desinfektion der Desinfektionsmittelbehälter höher angeordnet als die maximal mögliche Flüssigkeitssäule im Vorlagetank. In einer Leitung zwischen dem Desinfektionsmittelbehälters und dem Vorlagetank ist dazu vorteilhafterweise ein Magnetventil angeordnet, welches geöffnet wird, um Desinfektionsmittel in den Vorlagetank zu leiten. Die Ausleitungsvorrichtung ist dabei insbesondere durch das Magnetventil realisiert.

Bei beiden Ausführungen kann ein Überlaufbehälter vorgesehen sein, in welchen das Desinfektionsmittel geleitet wird, bevor es in den Vorlagetank geleitet wird.

In einer bevorzugten Ausführung des Verfahrens wird die Temperatur des Desinfektionsmittels und/oder die Temperatur des Desinfektionsmittelgemischs, d.h. des Gemisches aus Desinfektionsmittel und Wasser, geregelt. Neben dem rein chemischen Verfahren können dazu ein Heizgerät und eine Temperaturregelung in das Verfahren eingebunden werden. Dabei neben der Konzentration auch die Temperatur des Desinfektionsmittels und/oder Desinfektionsgemischs geregelt. Das Heizgerät bzw. der Heizer kann dabei zur Temperaturregelung des Desinfektionsmittels bzw. Desinfektionskonzentrates entweder in der Umkehrosmoseanlage, bevorzugterweise im Vorlagetank, angeordnet sein und/oder zur Regelung des Desinfektionsmittelgemischs extern an die Umkehrosmoseanlage/Ringleitung angeschlossen werden. Die Temperatur wird über wenigstens einen Temperatursensor geregelt. Der Temperatursensor ist bevorzugt im Vorlagetank angeordnet. Die Regelung erfolgt entweder zentral, insbesondere durch eine Steuerungseinheit oder wird von der Steuerungseinheit an eine Heizungssteuerungseinheit übertragen.

In Bezug auf die Umkehrosmoseanlage wird die oben genannte Aufgabe erfindungsgemäß gelöst durch eine Umkehrosmoseanlage mit den Merkmalen von Anspruch 14. Die Umkehrosmoseanlage weist dazu einen Vorlagetank, eine Ringleitung zum Anschluss von Verbrauchern, wenigstens ein Membranmodul, eine Zulaufleitung zum Membranmodul, wenigstens eine Druckpumpe zum Pumpen von Flüssigkeit in das Membranmodul sowie einen Desinfektionsmittelbehälter auf. Dabei sind eine Steuerungseinheit und Mittel zur Durchführung eines oben beschriebenen Verfahrens vorgesehen. Die Mittel können beispielsweise Ventile und Leitungen umfassen, um die beschriebenen Ströme von Desinfektionsmittel und Prozesswasser zu ermöglichen und welche von der Steuerungseinheit ansteuerbar sind. Das Verfahren kann in der Steuerungseinheit software- und/oder hardwaremäßig implementiert sein.

Vorteilhafterweise weist die Steuerungseinheit eine Schnittstelle zum Empfang und/oder Senden von Daten auf. Die Steuerungseinheit ist dabei vorteilhafterweise ertüchtigt, mithilfe eines Datenübertragungsprotokolls mit anderen Geräten zu kommunizieren. Dabei können Informationen wie Zielkonzentration und/oder Zieltemperatur von Verbrauchern, insbesondere den Dialysegeräten, angefordert werden und entsprechend für das Verfahren eingestellt werden. Diese Daten können auch zurück an die Dialysegeräte gesendet werden.

Die Steuerungseinheit kann auf diese Weise entweder die kleinste gemeinsame Konzentration und/oder Temperatur aller angeschlossenen Dialysegeräte erzeugen oder eine gestaffelte Abnahme ermöglichen. Bei einer gestaffelten Abnahme werden ähnliche Profile (Temperatur und/oder Konzentration) gebündelt und den entsprechenden Geräten erlaubt oder es wird verweigert, die Desinfektionsmittelmischung aus der Ringleitung abzunehmen.

Die Umkehrosmoseanlage weist vorteilhafterweise wenigstens eine Zirkulationspumpe zur Rückförderung von Konzentrat aus dem Membranmodul in die Zulaufleitung auf.

Die Vorteile der Erfindung liegen insbesondere darin, dass durch das beschriebene Verfahren eine Umkehrosmoseanlage mit angeschlossener Ringleitung inkl. der angeschlossenen Dialysegeräte desinfiziert werden kann. Dabei hat der Anwender insbesondere den Vorteil, dass das Desinfektionsmittel nicht mehr manuell dosiert werden muss. Außerdem kann der Anwender die vom Desinfektionsmittelherstellervorgegebene Konzentration und Zeit als Parameter übergeben und muss sich anschließend um keine weitere Aktion mehr kümmern. Dies sorgt für eine zeitliche Entlastung des Personals, sowie einen reproduzierbaren Desinfektionsablauf, bei dem keine Chemikalien verschwendet werden.

Ein Ausführungsbeispiel der Erfindung wird anhand einer Zeichnung näher erläutert. Darin zeigen in stark schematisierter Darstellung

FIG. 1    eine Umkehrosmoseanlage in einer ersten bevorzugten Ausführungsform;

FIG. 2    einen zylindrischen Vorlagetank;

FIG. 3    ein Ablaufplan eines Verfahrens in einer bevorzugten Ausführungsform;

FIG. 4     eine Umkehrosmoseanlage in einer zweiten bevorzugten Ausführungsform;

FIG. 5     eine Umkehrosmoseanlage in einer dritten bevorzugten Ausführungsform, und

FIG. 6     eine Umkehrosmoseanlage in einer vierten bevorzugten Ausführungsform.

Gleiche Teile sind in allen Figuren mit denselben Bezugszeichen versehen.

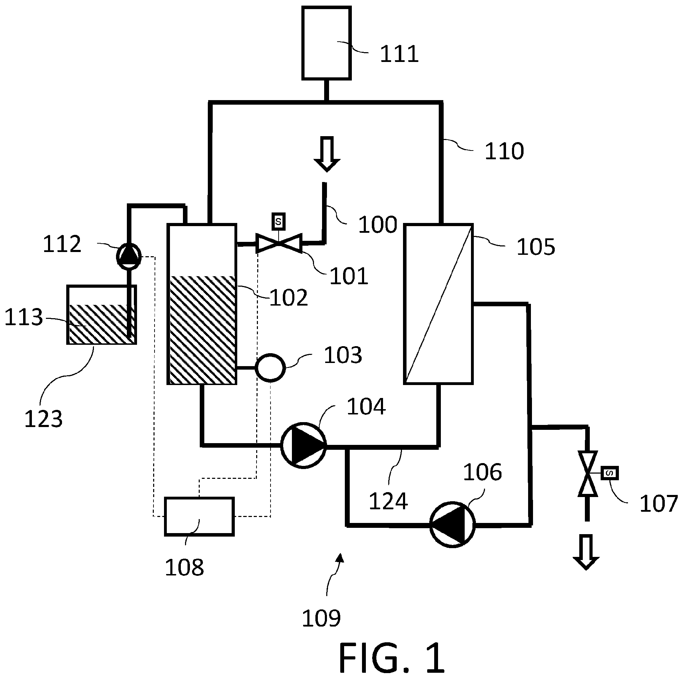

In FIG. 1 ist eine Umkehrosmoseanlage 109 dargestellt, welche an eine Ringleitung 110 angeschlossen ist. An der Ringleitung 110 sind an Abnahmestellen Verbraucher, vorliegend Dialysegeräte 111, angeschlossen.

Während des normalen Betriebes stellt die Umkehrosmoseanlage 109 Permeat her. Dazu wird Prozesseingangswasser 100 über ein geöffnetes Magnetventil 101 in einen Vorlagetank 102 geleitet. Das Prozesseingangswasser 100 wird über eine Druckpumpe 104 durch eine Zulaufleitung 124 in ein Membranmodul 105 geleitet. Ein erster Teil des Volumenstroms, nämlich das Konzentrat, wird entweder über eine Zirkulationspumpe 106 wieder in den Prozess eingebracht oder über ein Magnetventil 107 aus dem System herausgeleitet. Der andere Teil des Volumenstroms, nämlich das Permeat, wird in die Ringleitung 110 gespeist. Die an der Ringleitung 110 angeschlossenen Dialysegeräte 111 nehmen das Permeat für die Dialysebehandlung ab. Das nicht abgenommene Volumen wird zurück in den Vorlagetank 102 geleitet.

Die Umkehrosmoseanlage 109 weist eine Ausleitungsvorrichtung auf, welche als Dosierpumpe 112 ausgebildet ist, um Desinfektionsmittel 113 aus einem Desinfektionsmittelbehälter 123 in den Vorlagetank 102 zu pumpen.

Die Steuerungseinheit 108 ist signaleingangsseitig mit dem Drucksensor 103 und signalausgangsseitig mit der Dosierpumpe 112 und dem Magnetventil 101 verbunden.

In einem Verfahren zur chemischen Desinfektion wertet die Steuerungseinheit 108 kontinuierlich den Drucksensor 103 aus. Aufgrund der bekannten Geometrie des Vorlagetanks 102 und den ausgewerteten Druck des Drucksensors 103 wird das aktuelle Tankvolumen des Vorlagetanks 102 in Litern berechnet. Dadurch können Prozesseingangswasser 100 Desinfektionsmittel 113 im gewünschten Verhältnis in den Vorlagetank 102 eingebracht werden.

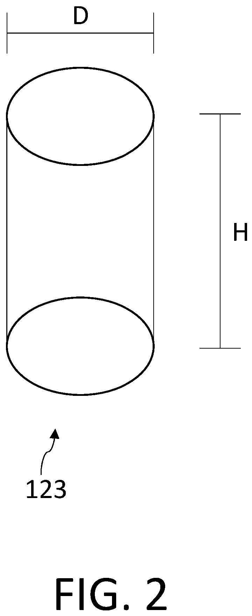

In FIG. 2 ist schematisch ein Vorlagetank 102 mit einem zylinderförmigen Querschnitt dargestellt, welcher einen Durchmesser D und eine Höhe H aufweist, sodass sich aus diesen Größen in bekannter Weise das Volumen des Vorlagetanks 102 berechnen lässt. Diese Ausgestaltung des Vorlagetanks 102 ist nur beispielhaft. Die tatsächliche Ausgestaltung der geometrischen Form des Vorlagetanks 102 (rund, eckig etc.) ist für das Verfahren nicht ausschlaggebend.

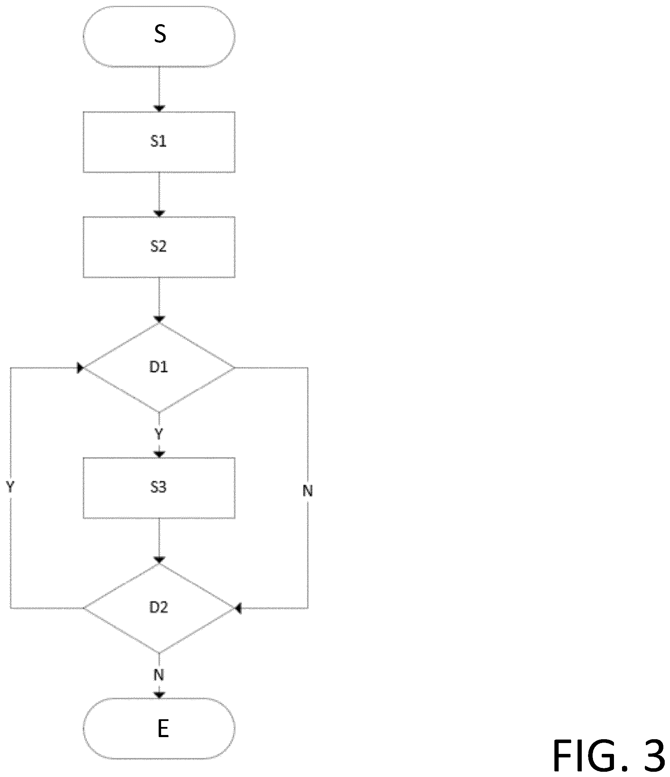

In FIG. 3 ist ein Ablaufdiagramm eines Verfahrens zur chemischen Desinfektion in einer bevorzugten Ausführungsform dargestellt, das in einem Start S beginnt.

In einer ersten Sequenz S1 startet ein Benutzer den Desinfektionsbetrieb und übergibt Verfahrensdaten an die Steuerungseinheit 108 über eine Benutzerschnittstelle (nicht dargestellt), welche insbesondere als GUI (Graphical User Interface) ausgebildet ist. Die Verfahrensdaten bzw. Einstellungen beinhalten dabei zumindest die gewünschte Zielkonzentration des Desinfektionsmittels. Zusätzlich zu der gewünschten Zielkonzentration Information werden zur Durchführung des Verfahrens das jeweilige Wasservolumen der Umkehrosmoseanlage 109 und der Ringleitung 110 benötigt. Die Steuerungseinheit 108 ist derart ausgebildet bzw. programmiert, dass in ihr diese Angaben hinterlegt sind. Das Volumen der Ringleitung 110 kann entweder direkt (in Liter) oder indirekt über den Rohrinnendurchmesser und die Ringleitungslänge angegeben werden. Da es sich bei diesen Werten eher um Parameter handelt, da sich das System nicht spontan und/oder häufig ändert, können diese Werte auch von autorisiertem Personal voreingestellt/ eingespeichert werden. In der Sequenz S1 wird weiter der Betriebsmodus der Umkehrosmose 109 für die Desinfektion gestartet.

Nach dem Starten des Betriebsmodus und der Eingabe der Verfahrensdaten bzw. Betriebsdaten in der Sequenz S1 wird in einer Sequenz S2 die benötigte Menge an Desinfektionsmittel berechnet. Die Berechnung erfolgt gemäß folgender Gleichung:

$$V_{Desinfektionsmittel}\ [l] = (\text{Zielkonzentration }[\%]/(1 - \text{Zielkonzentration }[\%])) * V_{Gesamtvolumen}\ [l].$$

Dabei bezeichnet $V_{Desinfektionsmittel}$ [l] das benötigte Volumen des Desinfektionsmittels in der Einheit Liter, Zielkonzentration [%] die Zielkonzentration des Desinfektionsmittels in Prozent, und $V_{Gesamtvolumen}$ [l] das Systemvolumen, d.h. die Summe der Volumina der Umkehrosmoseanlage 109 und der Ringleitung 110 in Litern. Das Symbol "*" steht für die Multiplikation.

Als Beispiel soll die Umkehrosmoseanlage 109 mit angeschlossener Ringleitung 110 bei einer Konzentration von 3% desinfiziert werden, wobei das Gesamtvolumen 100 Liter beträgt. Dazu ergibt sich das Volumen des Desinfektionsmittels zu (0,03/(1-0,03)) * 97 Liter = 3 Liter.

Die entsprechende Mischung aus Wasser und Desinfektionsmittel wird als Desinfektionsgemisch bezeichnet.

Um die Desinfektion zu beginnen, startet die Steuerungseinheit 108 die Druckpumpe 104 und die Zirkulationspumpe 106. Danach wird eine Wassermenge von 3 Litern Wasser aus dem Anlagenverbund, d.h. dem Gesamtsystem aus Umkehrosmoseanlage 109 und Ringleitung 110 ausgeleitet, indem die Steuerungseinheit 108 das Magnetventil 107 öffnet. Diese Ausleitung ist optional bzw. erfolgt nicht, wenn das Tankvolumen groß genug ist und dementsprechend kein Desinfektionsmittel überlaufen würde. Nachdem das Volumen entfernt wurde, werden die 3 Liter Wasser durch Desinfektionsmittel 113 ersetzt, welches mittels der Dosierpumpe 112 in den Vorlagetank 102 befördert wird.

Das Volumen von Flüssigkeit im Vorlagetank 102 wird mit Hilfe des Drucksensors 103 bestimmt. Vorliegend hat der in FIG. 2 dargestellte Vorlagetank 102 einen Durchmesser D von 0,30 m und eine Höhe H von 0,50 m.

Das Tankvolumen des Vorlagetanks ergibt sich dabei zu

$$\pi * (0{,}15 \text{ m})^2 * 0{,}5 \text{ m} = 0.0353 \text{ m}^3 = 35.34 \text{ Liter}.$$

Bei einer Annahme, dass eine Wassersäule von 1 m ungefähr 98,07 mBar Druck entspricht, kann jeder Liter Wasser mit 2.775 mBar bemessen werden. Im oberen Beispiel muss demnach der Startdruck um 8.325 mBar gesenkt werden und anschließend mit Desinfektionsmittel wieder auf den Startwert gebracht werden, um die gewünschte Konzentration zu erreichen.

Nachdem die initiale Konzentration in der Sequenz S2 durchgeführt wurde, wird über den gesamten Desinfektionszeitraum das Tankvolumen des Desinfektionsmittelbehälters 123 überwacht. In einer Entscheidung D1 wird überprüft, ob sich das Volumen von Desinfektionsmittel in dem Vorlagetank 102 verringert. Dies ist der Fall, wenn das wenigstens eine Dialysegerät 111 Desinfektionsmittel abnimmt. In diesem Fall verzweigt das Verfahren zu einer Sequenz S3.

In der Sequenz S3 erfolgt ein Nachfüllen des Desinfektionsgemischs. Das entnommene Desinfektionsmittelgemisch wird ersetzt. Dazu werden Prozesseingangswasser 100 über das Magnetventil 101 und Desinfektionsmittel 113 über die Dosierpumpe 112 nacheinander in der entsprechenden Menge in den Vorlagetank 102 gegeben (siehe auch Sequenz S2). Dieser Vorgang muss nicht kontinuierlich durchgeführt werden und kann erfolgen, sobald eine Mindestmenge, beispielsweise 5 Liter, abgenommen wurden. Dies hat den Vorteil, dass die Messunsicherheiten sowie Trägheit von Ventilen und Pumpen besser beherrschbar sind. Der Vorlagetank 102 wird immer nur zur selben Zeit mit einer Flüssigkeit gefüllt.

In einer Entscheidung D2 wird überprüft, ob die Desinfektion der Umkehrosmoseanlage 109 noch durchgeführt wird. Solange die Desinfektion durchgeführt wird bzw. aktiv ist, wird die oben genannte Konzentrationsregelung durchgeführt und das Verfahren verzweigt wieder zur Entscheidung D1, ansonsten endet es in einem Ende E. Sobald der Nutzer oder die Steuerungseinheit 108 den Betriebsmodus bzw. die Betriebsphase wechselt, wird bevorzugt die oben beschriebene Konzentrationsregelung durchgeführt.

Falls in der Entscheidung D1 festgestellt wird, dass sich das Volumen von Desinfektionsmittel in dem Desinfektionsmittelbehälter 123 nicht verringert, verzweigt das Verfahren von dort zu der Entscheidung D2.

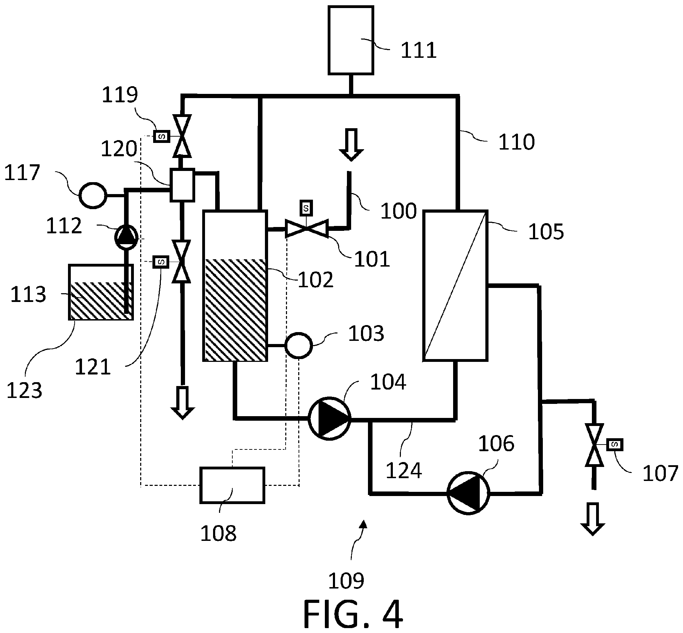

Eine Umkehrosmoseanlage 109 in einer weiteren bevorzugten Ausführungsform für das beschriebene Verfahren ist in FIG. 4 dargestellt. Die Umkehrosmoseanlage 109 weist zwei Magnetventile 119 und 121 auf sowie einen Überlaufbehälter 120, der hydraulisch zwischen den Dosiermitteltank 123 und den Vorlagetank 102 geschaltet ist. Das Magnetventil 119 ist hydraulisch zwischen den Überlaufbehälter 120 und die Ringleitung 110 geschaltet. Das Magnetventil 121 ist eine Leitung zur Abführung von Flüssigkeit aus dem Überlaufbehälter 120 in einen Abfluss geschaltet, sodass durch Öffnen des Magnetventils 121 der Überlaufbehälter 120 geleert werden kann.

Die Magnetventile 119 und 121 sind nie bzw. werden nie gleichzeitig geöffnet oder geschlossen. Eines der beiden Magnetventile 119, 121 ist immer offen, während das entsprechend andere Magnetventil 121, 119 geschlossen ist. Die Steuerung des jeweiligen Schaltzustandes der beiden Magnetventile 119, 121 erfolgt durch die Steuerungseinheit 108. Wenn Bedarf an Desinfektionsmittel besteht, wird die Dosierpumpe 112 aktiviert, sodass sie Desinfektionsmittel 113 in den Überlaufbehälter 120 pumpt. Das Volumen an Desinfektionsmittel wird über einen Volumenstromsensor 117 erfasst. Zusammen mit dem Permeat aus der Ringleitung 110 wird der Überlaufbehälter 120 über das geöffnete Magnetventil 119 so lange gefüllt, bis das Volumen in den Vorlagetank 120 überläuft. Nachdem die Desinfektion beendet wurde, wird das Magnetventil 119 geschlossen und das Magnetventil 121 geöffnet. Das restliche Desinfektionsmittel 113, welches sich im Überlauftank 120 befindet, wird in den Abfluss geleitet. Die Ausleitungsvorrichtung ist bei dieser Ausführungsform durch die Dosierpumpe 112 gegeben.

Sollte die Dosierpumpe 112 nicht aufhören, Desinfektionsmittel 113 zu fördern, würde dies aufgrund des geöffneten Magnetventils 121 und des höher liegenden Überlaufbehälters 120 nicht in den Vorlauftank 102 gelangen. Zum Ausspülen des Überlaufbehälters 120 kann das Magnetventil 119 geöffnet werden, ohne die Dosierpumpe 112 anzuschalten.

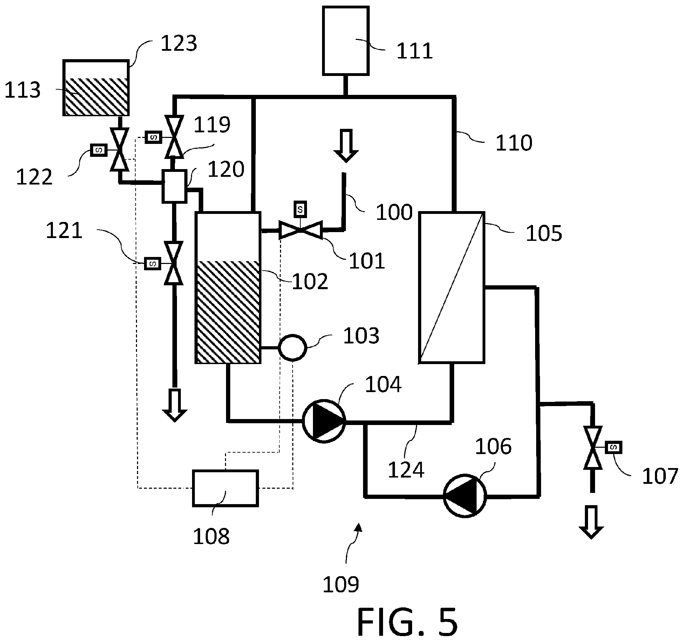

In FIG. 5 ist eine Umkehrosmoseanlage 109 in einer weiteren bevorzugten Ausführungsform für das beschriebene Verfahren dargestellt, welche keine Dosierpumpe 112 für das Desinfektionsmittel aufweist. Für den Transport des Desinfektionsmittels 113 in den Vorlagetank 102 wird die Schwerkraft ausgenutzt. Dazu ist im montierten Zustand der Umkehrosmoseanlage 109 der Desinfektionsmittelbehälter 123 höher montiert als der Vorlagetank. Die Umkehrosmoseanlage 109 weist die zwei Magnetventile 119, 121 und den Überlaufbehälter 120 auf. Durch ein Magnetventil 122 wird der Ablauf von Desinfektionsmittel aus dem Desinfektionsmittelbehälter 123 ermöglicht. Die Ausleitungsvorrichtung ist hierbei durch das Magnetventil 122 realisiert.

Bei geöffnetem Magnetventil 122 läuft Desinfektionsmittel erst in den Überlauftank 120 und anschließend in den Vorlagetank 102. Bei dieser Umkehrosmoseanlage 109 bleiben die Magnetventile 119, 121 für die Dauer der Desinfektion geschlossen. Nachdem kein Desinfektionsmittel mehr benötigt wird, wird der Überlaufbehälter 120 zusammen mit den in den Vorlagetank 102 führenden Bereiche ausgespült, indem das Magnetventil 119 geöffnet wird. Nach beendetem Ausspülen und bis zur nächsten Desinfektion wird das Magnetventil 119 geschlossen und das Magnetventil 121 wird geöffnet. Sollte es zu einer fehlerhaften Stellung des Magnetventils 122 kommen, kann das Desinfektionsmittel über das Magnetventil 121 in den Abfluss fließen.

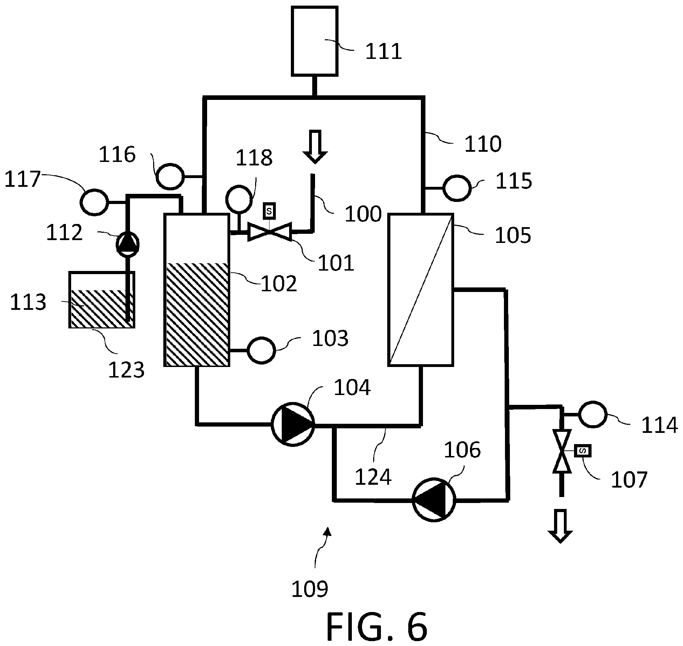

In FIG. 6 ist eine weitere bevorzugte Ausführungsform einer Umkehrosmoseanlage 109 für das Verfahren dargestellt. Diese Umkehrosmoseanlage 109 weist zwei Volumenstromsensoren 116, 117 auf. Der Volumenstromsensor 116 misst den Volumenstrom aus dem Vorlagetank 102 in die Ringleitung 110. Der Volumenstromsensor 117 misst den Volumenstrom aus dem Desinfektionsmittelbehälter 123 in den Vorlagetank 102. Durch das direkte Messen der beiden Volumenströme kann bei bekanntem Ringleitungsrücklauf die Menge des Inhalts bzw. des abgeflossenen Inhalts in dem Vorlagetank bzw. Desinfektionsmittel 123 durch zeitliche Integration direkt bestimmt werden. Die Dosierpumpe 112 ist hierbei die Ausleitungsvorrichtung.

Bezugszeichenliste

| | |
|---|---|
| 100 | Prozesseingangswasser |
| 101 | Magnetventil |
| 102 | Vorlagetank |
| 103 | Drucksensor |
| 104 | Druckpumpe |
| 105 | Membranmodul |
| 106 | Zirkulationspumpe |
| 107 | Magnetventile |
| 108 | Steuerungseinheit |
| 109 | Umkehrosmoseanlage |
| 110 | Ringleitung |
| 111 | Dialysegerät |
| 112 | Dosierpumpe |
| 113 | Desinfektionsmittel |
| 114 | Volumenstromsensor |
| 115 | Volumenstromsensor |
| 116 | Volumenstromsensor |
| 117 | Volumenstromsensor |
| 118 | Volumenstromsensor |
| 119 | Magnetventil |
| 120 | Überlaufbehälter |
| 121 | Magnetventil |
| 122 | Magnetventil |
| 123 | Desinfektionsmittelbehälter |
| 124 | Zulaufleitung |
| D | Durchmesser |
| H | Höhe |
| S | Start |
| E | Ende |
| S1 | Sequenz |
| S2 | Sequenz |
| S3 | Sequenz |
| D1 | Entscheidung |
| D2 | Entscheidung |

**Patentansprüche**

1. Verfahren zur chemischen Desinfektion einer Umkehrosmoseanlage (109), welche einen Vorlagetank (102), eine Ringleitung (110) zum Anschluss von Verbrauchern (111), wenigstens ein Membranmodul (105), eine Zulaufleitung (124) zum Membranmodul (105), wenigstens eine Druckpumpe (104) zum Pumpen von Flüssigkeit in das Membranmodul (105) sowie einen Desinfektionsmittelbehälter (123) umfasst, wobei Desinfektionsmittel aus dem Desinfektionsmittelbehälter (123) in den Vorlagetank (102) gefördert wird, **gekennzeichnet durch** die Schritte

   - Festsetzen einer Zielkonzentration des Desinfektionsmittels;
   - Bestimmen der aufgrund der Zielkonzentration des Desinfektionsmittels benötigten Menge des Desinfektionsmittels;
   - Ausleiten der für die Zielkonzentration benötigten Menge des Desinfektionsmittels aus dem Desinfektionsmittelbehälter (123) in den Vorlagetank (102) durch eine Ausleitungsvorrichtung (112, 122)
   - Überwachen und Bestimmen der aktuellen Konzentration des Desinfektionsmittels;
   - wenn die aktuelle Konzentration des Desinfektionsmittels sich von der Zielkonzentration im Rahmen eines Schwellenbereiches unterscheidet, Nachfördern von Desinfektionsmittel und Prozesseingangswasser in den Vorlagetank (102) entsprechend der Zielkonzentration,

   wobei das Überwachen und Bestimmen der aktuellen Konzentration des Desinfektionsmittels durch Bestimmen des Flüssigkeitsvolumens der Flüssigkeit im Vorlagetank (102) durchgeführt wird.

2. Verfahren nach Anspruch 1, wobei das Bestimmen des Flüssigkeitsvolumens durch eine Druckmessung mit einem Drucksensor (103), mechanisch, optisch und/oder elektrisch erfolgt.

3. Verfahren nach Anspruch 1, wobei das Bestimmen des Flüssigkeitsvolumens durch eine Volumenstrommessung wenigstens eines Volumenstromsensors (116) erfolgt.

4. Verfahren nach Anspruch 1, wobei das Bestimmen der aktuellen Konzentration mit wenigstens einem stoffselektiven Sensor durchgeführt wird.

5. Verfahren nach Anspruch 4, wobei der stoffselektive Sensor als amperometrischer Sensor ausgebildet ist.

6. Verfahren nach Anspruch 1, wobei das Bestimmen der aktuellen Konzentration des Desinfektionsmittels durch eine pH-Wert-Messung durchgeführt wird.

7. Verfahren nach einem der Ansprüche 1 bis 6, wobei das Nachfördern von Desinfektionsmittel und Prozesseingangswasser in den Vorlagetank (102) kontinuierlich erfolgt.

8. Verfahren nach einem der Ansprüche 1 bis 6, wobei das Nachfördern erfolgt, wenn die dem Vorlagetank (102) entnommene Flüssigkeitsmenge einen Volumenschwellenwert überschreitet.

9. Verfahren nach einem der Ansprüche 1 bis 8, wobei das Fördern von Desinfektionsmittel aus dem Desinfektionsmittelbehälter (123) in den Vorlagetank (102) mit einer Dosierpumpe (112) als Ausleitvorrichtung durchgeführt wird.

10. Verfahren nach einem der Ansprüche 1 bis 8, wobei das Fördern von Desinfektionsmittel aus dem Desinfektionsmittelbehälter (123) in den Vorlagetank (102) durch Nutzung der Schwerkraft erfolgt.

11. Verfahren nach einem der vorherigen Ansprüche, wobei die Temperatur des Desinfektionsmittels und/oder des Desinfektionsmittelgemisches geregelt wird.

12. Umkehrosmoseanlage (109), umfassend einen Vorlagetank (102), eine Ringleitung (110) zum Anschluss von Verbrauchern (111), wenigstens ein Membranmodul (105), eine Zulaufleitung (124) zum Membranmodul (105), wenigstens eine Druckpumpe (104) zum Pumpen von Flüssigkeit in das Membranmodul sowie einen Desinfektionsmittelbehälter (123) umfasst, **gekennzeichnet durch** eine Steuerungseinheit (108) und Mittel zur Durchführung eines Verfahrens nach einem der vorherigen Ansprüche.

13. Umkehrosmoseanlage (109) nach Anspruch 12, wobei die Steuerungseinheit (109) eine Schnittstelle zum Empfang und/oder Senden von Daten aufweist.

FIG. 1

FIG. 2

FIG. 3

FIG. 4

FIG. 5

FIG. 6

Europäisches
Patentamt

European
Patent Office

Office européen
des brevets

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | DE 10 2006 026107 B3 (VOELKER MANFRED [DE]) 13. Dezember 2007 (2007-12-13)<br><br>* Absätze [0004], [0021] - [0023]; Abbildungen 1, 2 *<br>----- | 1,2, 4-10,12, 13 | INV.<br>C02F1/00<br>C02F1/44<br>C02F1/68<br>B01D61/02<br>B01D61/12 |
| X | DE 10 2014 017404 A1 (FIDICA GMBH & CO KG [DE]) 25. Mai 2016 (2016-05-25)<br>* Absätze [0035] - [0037], [0058], [0069] - [0070], [0077], [0081]; Abbildungen 1, 2 *<br>----- | 1-13 | B01D65/02<br><br>ADD.<br>C02F1/72 |
| X | EP 2 674 399 A1 (VOELKER MANFRED [DE]) 18. Dezember 2013 (2013-12-18)<br><br>* Absätze [0005], [0012], [0013]; Abbildungen 1, 2 *<br>----- | 1-4,6, 8-10,12, 13 | |

**RECHERCHIERTE SACHGEBIETE (IPC)**

C02F
B01D

Der vorliegende Recherchenbericht wurde für alle Patentansprüche erstellt

| Recherchenort | Abschlußdatum der Recherche | Prüfer |
|---|---|---|
| Den Haag | 30. September 2025 | Wolf, Gundula |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
.....................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 25 17 4539

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-09-2025

| Im Recherchenbericht angeführtes Patentdokument | Datum der Veröffentlichung | Mitglied(er) der Patentfamilie | | Datum der Veröffentlichung |
|---|---|---|---|---|
| DE 102006026107 B3 | 13-12-2007 | AT | E448864 T1 | 15-12-2009 |
| | | AU | 2007202471 A1 | 20-12-2007 |
| | | DE | 102006026107 B3 | 13-12-2007 |
| | | DK | 1862213 T3 | 06-04-2010 |
| | | EP | 1862213 A1 | 05-12-2007 |
| | | ES | 2336955 T3 | 19-04-2010 |
| | | PL | 1862213 T3 | 31-05-2010 |
| | | PT | 1862213 E | 23-02-2010 |
| | | SI | 1862213 T1 | 30-07-2010 |
| DE 102014017404 A1 | 25-05-2016 | DE | 102014017404 A1 | 25-05-2016 |
| | | EP | 3056257 A1 | 17-08-2016 |
| | | US | 2017021308 A1 | 26-01-2017 |
| EP 2674399 A1 | 18-12-2013 | BR | 102012032456 A2 | 22-04-2015 |
| | | CN | 103482725 A | 01-01-2014 |
| | | EP | 2674399 A1 | 18-12-2013 |
| | | ES | 2488632 T3 | 28-08-2014 |
| | | PL | 2674399 T3 | 28-11-2014 |
| | | RU | 2012134683 A | 20-02-2014 |
| | | US | 2013334115 A1 | 19-12-2013 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19538818 A1

- DE 102006026107 B3